# EUROPÄISCHE PATENTSCHRIFT

(11) **EP 2 621 427 B1**
(45) Veröffentlichungstag und Bekanntmachung des Hinweises auf die Patenterteilung: **26.10.2016**
(21) Anmeldenummer: 10768400.3
(22) Anmeldetag: 30.09.2010
(51) Int. Cl.: A61F 9/009

(54) **SCHNITTSTELLENEINHEIT ZUR POSITIONIERUNG EINES BESTRAHLUNGSOBJEKTS GEGENÜBER EINER STRAHLUNGSQUELLE**
INTERFACE UNIT FOR POSITIONING AN IRRADIATION OBJECT RELATIVE TO A RADIATION SOURCE
UNITÉ D'INTERFACE POUR LE POSITIONNEMENT D'UN OBJET D'IRRADIATION PAR RAPPORT À UNE SOURCE DE RAYONNEMENT

(43) Veröffentlichungstag der Anmeldung: 07.08.2013
(73) Patentinhaber: WaveLight GmbH, 91058 Erlangen (DE)
(72) Erfinder: VOGLER, Klaus, 90542 Eckental/Eschenau (DE); DEISINGER, Thomas, 90556 Cadolzburg (DE); ROBL, Gerhard, 90547 Stein (DE)
(74) Vertreter: Katérle, Axel
(86) Internationale Anmeldenummer: PCT/EP2010/005972
(87) Internationale Veröffentlichungsnummer: WO 2012/041347

(56) Entgegenhaltungen:
- EP-A1- 1 891 915
- EP-A2- 1 844 745
- WO-A1-2010/022745
- DE-A1- 10 052 068
- DE-A1-102006 056 711
- US-A1- 2002 103 481
- US-A1- 2007 093 795

## Beschreibung

Die Erfindung betrifft eine Schnittstelleneinheit zur Positionierung eines Bestrahlungsobjekts gegenüber einer Strahlungsquelle, wobei die Schnittstelleneinheit mindestens eine erste Positionierungsfläche zur Positionierung der Schnittstelleneinheit gegenüber der Strahlungsquelle sowie eine zweite Positionierungsfläche zur Anlage an dem Bestrahlungsobjekt aufweist, wobei die Schnittstelleneinheit einen durch die zweite Positionierungsfläche hindurch verlaufenden Weg für die Strahlung der Strahlungsquelle bereitstellt.

Eine solche Schnittstelleneinheit kann beispielsweise bei der laserchirurgischen Schneidbearbeitung des humanen Auges zum Einsatz kommen. Solche Schnittstelleneinheiten sind beispielsweise aus EP1844745, US2007/0093795 oder US2002/0103481 bekannt. Die Schnittstelleneinheit, die man im Fall eines menschlichen Bestrahlungsobjekts auch als Patienteninterface bezeichnen kann, dient in diesem Fall zur Herstellung einer positionsfesten Kopplung zwischen dem Patientenauge und dem die Laserstrahlung bereitstellenden Lasersystem, üblicherweise zwischen dem Auge und einem Fokussierobjektiv des Lasersystems. Die feste Kopplung ist nötig, um den Abstand des Fokussierobjektivs von dem Patientenauge konstant zu halten, damit der Schnitt mit der gewünschten hohen Präzision in dem zu bearbeitenden Gewebestück des Auges, beispielsweise der Kornea, gesetzt werden kann.

Zur Schneidbearbeitung nicht nur von Augengewebe sondern auch von anderem biologischen Gewebe und auch von toter Materie ist die Nutzung des sogenannten laserinduzierten optischen Durchbruchs an sich bekannt. Ein solcher Durchbruch entsteht bei Einstrahlung fokussierter Laserstrahlung im Bereich des Fokus bei hinreichender, über der Durchbruchsschwelle liegenden örtlichen und zeitlichen Energiedichte der Pulse. Der optische Durchbruch bewirkt eine weitestgehend athermische, lokale Zerstörung des behandelten Gewebes. Dieser Effekt wird als Photodisruption bezeichnet. Durch Aneinanderreihung solcher Photodisruptionen kann in dem bearbeiteten Gewebe eine nahezu beliebige dreidimensionale Schnittfigur erzeugt werden. Typischerweise werden heutzutage Lasersysteme mit Pulsdauern der Laserstrahlung in Femtosekundenbereich für die Schneidbearbeitung eingesetzt. Bei diesen ultrakurzen Pulsdauern ist die Durchbruchsschwelle vergleichsweise niedrig, was für eine niedrige Strahlungsbelastung des behandelten Gewebes günstig ist. Die Größe der Photodisruption ist im wesentlichen auf die Ausdehnung des Strahlungsfokus begrenzt. Die Schneidpräzision hängt somit entscheidend von der örtlichen Einstellgenauigkeit des Fokus ab.

Zwar ist durch eine Schnittstelleneinheit der hier betrachteten Art eine Referenzierung der Augenvorderfläche gegenüber dem Koordinatensystem des Lasersystems möglich. Allerdings bewirken Fertigungstoleranzen der Schnittstelleneinheit, die sich in Toleranzen der optischen Eigenschaften der Schnittstelleneinheit niederschlagen können, entsprechende Abweichungen der Fokusposition im zu behandelnden Gewebe. Die Schneidpräzision hängt damit entscheidend von der Fertigungspräzision der Schnittstelleneinheit ab. Eine hohe Fertigungsgenauigkeit der Schnittstelleneinheit ist daher sehr erstrebenswert.

Die Schnittstelleneinheit kann beispielsweise ein hülsenartiges Abstandsstück und ein an einer Stirnseite angeordnetes optisches Fenster aufweisen, wobei die Laserstrahlung längs der Hülsenachse durch den Innenbereich des Abstandsstücks hindurchgeht, das Fenster durchläuft und sodann aus der Schnittstelleneinheit austritt. Die Außenseite des Fensters dient zur Anlage des zu behandelnden Objekts, beispielsweise des zu behandelnden Auges. Die Laserstrahlung tritt somit aus dem Fenster unmittelbar in das zu behandelnde Material über. Dementsprechend bildet die Außenseite des Fensters eine Positionierungsfläche für die Positionierung des Bestrahlungsobjekts. Am fensterfernen Hülsenende ist die Schnittstelleneinheit zudem mit geeigneten Positionierformationen zur axialen Positionierung der Schnittstelleneinheit gegenüber dem Lasersystem ausgestattet. Das Abstandstück kann beispielsweise zylinderhülsenförmig ausgebildet sein; im Stand der Technik sind Lösungen bekannt, bei denen für das Abstandsstück eine Kegelhülsenform gewählt ist, wobei das Fenster am schmalen Kegelende vorgesehen ist. Das Abstandsstück kann bei diesen Lösungen als Abstandskegel bezeichnet werden; es versteht sich, dass im Rahmen der Erfindung eine Kegelform für das Abstandsstück keineswegs zwingend ist.

Eine Möglichkeit zur Fertigung einer in obiger Weise mit einem Abstandsstück und einem Fenster für den Strahlungsdurchtritt ausgeführten Schnittstelleneinheit besteht darin, das Abstandsstück aus einem Metallwerkstoff, beispielsweise Aluminium, zu fertigen und für das Fenster eine den optischen Anforderungen genügende Glasplatte in eine Einfassung des Abstandsstücks einzusetzen und darin zu verkleben. Eine solche Fertigungsmethode stellt jedoch besonders hohe Anforderungen an die Einhaltung zulässiger Toleranzen, weil sich die individuellen Fertigungstoleranzen des Abstandsstücks und der Glassplatte addieren können und zudem der Klebevorgang eine weitere Quelle von Ungenauigkeiten sein kann.

Aufgabe der Erfindung ist es, eine Schnittstelleneinheit der eingangs bezeichneten Art mit hoher Genauigkeit herstellen zu können. Zur Lösung dieser Aufgabe schlägt die Erfindung vor, dass die Schnittstelleneinheit einen sowohl die mindestens eine erste Positionierungsfiläche als auch die zweite Positionierungsfläche bildenden, einstückig hergestellten Schnittstellenkörper umfasst. Dadurch können Montageungenauigkeiten entfallen, die ansonsten zu befürchten sind, wenn die mindestens eine erste Positionierungsfläche an einem ersten Teilkörper und die zweite Positionierungsfläche einem zweiten, gesondert gefertigten Teilkörper vorgesehen sind und die beiden Teilkörper miteinander zu verkleben oder anderweitig fest zu verbinden sind. Die bei Herstellung der Schnittstelleneinheit aus gesonderten Teilkörpern zu berücksichtigende Toleranzkette aus der Fertigungsgenauigkeit jedes einzelnen der Teilkörper sowie der Montagegenauigkeit der Verbindung der Teilkörper kann durch die erfindungsgemäße Lösung auf etwaige Fertigungsungenauigkeiten des einstückigen Schnittstellenkörpers reduziert werden. Eine Addition mehrerer Einzeltoleranzen kann vermieden werden. Dies gilt nicht nur für die geometrischen Abmessungen der Schnittstelleneinheit sondern auch für ihre optischen Eigenschaften (optische Weglänge).

Die mindestens eine erste Positionierungsfläche definiert eine strahlungseingangsseitige Referenzfläche der Schnittstelleneinheit, während die zweite Positionierungsfläche eines strahlungsausgangsseitige Referenzfläche definiert. Der von der Schnittstelleneinheit für die Strahlung bereitgestellte Weg verläuft in Richtung von der strahlungseingangsseitigen Referenzfläche zur strahlungsausgangsseitigen Referenzfläche. Längs dieses Wegs durchläuft die Strahlung mindestens zwei Medien unterschiedlicher optischer Dichte, wobei in einer einfachen und zweckmäßigen Ausgestaltung eines der Medien Luft und das andere Medium das Material eines die zweite Positionierungsfläche bildenden, für die Strahlung transparenten Fensterelements sein kann.

In einer bevorzugten Ausgestaltung ist der Schnittstellenkörper aus einem für ein Spritzverfahren, vorzugsweise ein Spritzprägeverfahren, geeigneten Material hergestellt, wobei es sich bei diesem Material vorzugsweise um ein Kunststoffmaterial handelt. Das Kunststoffmaterial kann beispielsweise ein Cyclo-Olefin-Copolymer, ein Cyclo-Olefin-Polymer, Polycarbonat oder Polymethylmethacrylat umfassen. Es versteht sich, dass dies rein beispielhafte Materialnennungen sind und andere spritzfähige, insbesondere biokompatible Kunststoffmaterialien möglich sind.

Vorzugsweise ist zumindest ein Teil des Schnittstellenkörpers im sichtbaren Wellenlängenbereich transparent. Eine solche Transparenz des Schnittstellenkörpers ist insbesondere für einen solchen Teil des Schnittstellenkörpers zweckmäßig, der ein den Strahlungsweg umschließendes Abstandsstück bildet. Die Transparenz ermöglicht es, partielle Abschattungen des Lichts einer zur Ausleuchtung des Operationsfelds verwendeten Lichtquelle zu vermeiden. Es ist dann sogar möglich, bei Ausgestaltung der Schnittstelleneinheit mit einem zylindrischen oder kegelförmigen Abstandsstück den Umfangsmantel desselben durchgehend zusammenhängend auszuführen, also frei von etwaigen Durchbrechungen.

Der gesamte Schnittstellenkörper kann gemäß einer Ausgestaltung aus demselben Werkstoff bestehen. Gemäß einer alternativen Ausgestaltung kann der Schnittstellenkörper verschiedene Bereiche besitzen, die aus jeweils unterschiedlichem Werkstoff bestehen. Dennoch wird der Schnittstellenkörper auch bei dieser alternativen Ausgestaltung als ein Stück hergestellt. Bei Vorhandensein verschiedener Bereiche des Schnittstellenkörpers, die aus unterschiedlichen Werkstoffen bestehen, ist eine einstückige Herstellung des Schnittstellenkörpers beispielsweise dadurch möglich, dass die verschiedenen Bereiche im selben Herstellungsschritt in einer Spritzform gemeinsam gespritzt werden. Moderne Mehrkomponenten-Spritzgussanlagen sind in der Lage, Bauteile aus verschiedenen Werkstoffen zu spritzen. Es ist überdies im Rahmen der Erfindung nicht ausgeschlossen, mindestens ein Teilstück des Schnittstellenkörpers vorzufertigen und die übrigen Bereiche des Schnittstellenkörpers sodann an das vorgefertigte Teilstück anzuspritzen, so dass sich eine stoffschlüssige Verbindung ergibt. Beispielsweise ist es vorstellbar, eine vorgefertigte Saugringkomponente zu verwenden, diese in eine Spritzform einzulegen und sodann die verbleibenden Bereiche des Schnittstellenkörpers an die Saugringkomponente anzuspritzen. Bevorzugt besteht jedenfalls zumindest ein die beiden Positionierungsflächen umfassender, zusammenhängender Abschnitt des Schnittstellenkörpers aus demselben Werkstoff.

In einer bevorzugten Ausgestaltung weist der Schnittstellenkörper einen den Weg für die Strahlung umschließenden Abstandskegel sowie ein am schmalen Ende des Abstandskegels vorgesehenes Kontaktelement zur Anlage an dem Bestrahlungsobjekt auf. Das Kontaktelement bildet das erwähnte Fenster für den Austritt der Strahlung. Es kann eine augenzugewandte Kontaktfläche aufweisen, die plan, konkav oder konvex ausgeführt ist oder abgerundete Randbereiche besitzt. Auf seiner der Strahlungsquelle zugewandten Seite (d.h. auf der augenabgewandten Seite) kann das Kontaktelement dagegen plan oder mit einer Freiformfläche ausgeführt sein. Beispielsweise kann als Kontaktelement eine biplanare Kontaktplatte dienen oder es kann ein plan-konkaves oder plan-konvexes Kontaktelement verwendet werden, bei dem die augenzugewandte Kontaktfläche konkav bzw. konvex ist und die gegenüberliegende, augenabgewandte Seite plan ausgeführt ist. Durch eine Gestaltung der augenabgewandten Seite des Kontaktelements als Freiformfläche ist es möglich, bei einer nichtplanen Ausgestaltung der Kontaktfläche eine durch Randverzeichnung (sphärische Aberrationen) bedingte Fokusverschlechterung auszugleichen. Besonders in einem Spritzverfahren sind an sich beliebige Gestaltungen einer solchen Freiformfläche realisierbar, so dass die Freiformfläche optimal im Hinblick auf die Kompensation etwaiger Aberrationen gestaltet werden kann, die durch eine nichtplane Ausführung der Kontaktfläche hervorgerufen werden können.

Statt einer biplanaren Kontaktplatte kann ein in anderer Weise anwendungsspezifisch gestaltetes Kontaktelement verwendet werden, beispielsweise ein plan-konkaves oder plan-konvexes Kontaktelement oder/und eines mit abgerundeten Randflächen. Die dem Bestrahlungsobjekt zugewandte Seite des Kontaktelements kann beispielsweise statt plan auch konvex sein oder am Rand abgerundet sein.

Das Kontaktelement kann auf seiner augenzugewandten Seite oder/und seiner augenabgewandten Seite mit einer reflexionsmindernden Beschichtung versehen sein, um etwaige Reflexionsverluste des Materials des Schnittstellenkörpers bei der Wellenlänge der verwendeten Strahlung zu verringern.

Es ist bekannt, Schnittstelleneinheiten mit der hier betrachteten Funktionalität zumindest bei ophthalmologischen Operationen in Verbindung mit einem Saugring einzusetzen, der zunächst auf das Auge aufgesetzt und dort durch Saugkraft fixiert wird. Anschließend wird die Schnittstelleneinheit an den Saugring angenähert und mit diesem in Eingriff gebracht. Dadurch wird nicht nur das Auge durch den Saugring fixiert, sondern auch die Schnittstelleneinheit gegenüber dem Saugring positioniert, insbesondere zentriert. In einer Ausgestaltung der Erfindung ist es nun vorstellbar, den Schnittstellenkörper mit einstückig angeformten Formationen auszuführen, die die Funktionalität eines solchen Saugrings erfüllen können. Dementsprechend kann der Schnittstellenkörper mindestens einen zum Bestrahlungsobjekt hin zumindest teilweise offenen Evakuierungsraum zur Saugfixierung des Schnittstellenkörpers an dem Bestrahlungsobjekt aufweisen. Auf die Verwendung eines gesonderten Saugrings kann dann verzichtet werden.

Es wurde bereits erläutert, dass der Schnittstellenkörper verschiedene Bereiche besitzen kann, die aus jeweils unterschiedlichem Werkstoff bestehen können. Dieser Gedanke kann bei einem Schnittstellenkörper, der mit Formationen ausgestattet ist, welche die Funktion eines herkömmlichen Saugrings erfüllen, dazu genutzt werden, einen die beiden Positionierungsflächen umfassenden, zusammenhängenden ersten Abschnitt des Schnittstellenkörpers aus einem anderen Werkstoff zu bilden als einen solche Saugringformationen (z. B. einen Evakuierungsraum) bildenden zweiten Abschnitt des Schnittstellenkörpers. Beispielsweise kann der zweite Abschnitt aus Macrolon oder einem anderen Kunststoff bestehen. Es ist sogar nicht ausgeschlossen, dass der zweite Abschnitt aus einem metallischen Werkstoff besteht.

Nach einem weiteren Gesichtspunkt betrifft die Erfindung ein Verfahren zur Herstellung einer Schnittstelleneinheit der vorstehend bezeichneten Art. Der Schnittstellenkörper wird dabei in einem Spritzprägeverfahren hergestellt. Beim Spritzprägen wird die Kunststoffschmelze in das noch nicht vollständig geschlossene Spritzwerkzeug eingespritzt. Erst während des Erstarrungsvorgangs wird das Werkzeug komplett geschlossen. Der sich dadurch aufbauende Schließdruck sorgt für die endgültige Ausformung des Formteils. Es handelt sich insoweit um ein kombiniertes Spritzguss-Druckformungs-Verfahren. Es hat sich gezeigt, dass mit einem Spritzprägeverfahren Kunststoff-Schnittstellenkörper in hoher Stückzahl mit der geforderten optischen Qualität zur einem vergleichsweise günstigen Preis und vor allem mit außerordentlich hoher Fertigungsgenauigkeit herstellbar sind.

Die Erfindung wird nachfolgend anhand der beigefügten Zeichnungen weiter erläutert. Es stellen dar:
- Figur 1: eine Schnittstelleneinheit nach einem Ausführungsbeispiel in teilweise aufgeschnittener Perspektivansicht,
- Figur 2: die Schnittstelleneinheit der Figur 1 zusammen mit einer auf einem humanen Auge platzierten Saugringeinheit und
- Figur 3: schematisch im Schnitt eine Schnittstelleneinheit nach einem weiteren Ausführungsbeispiel.

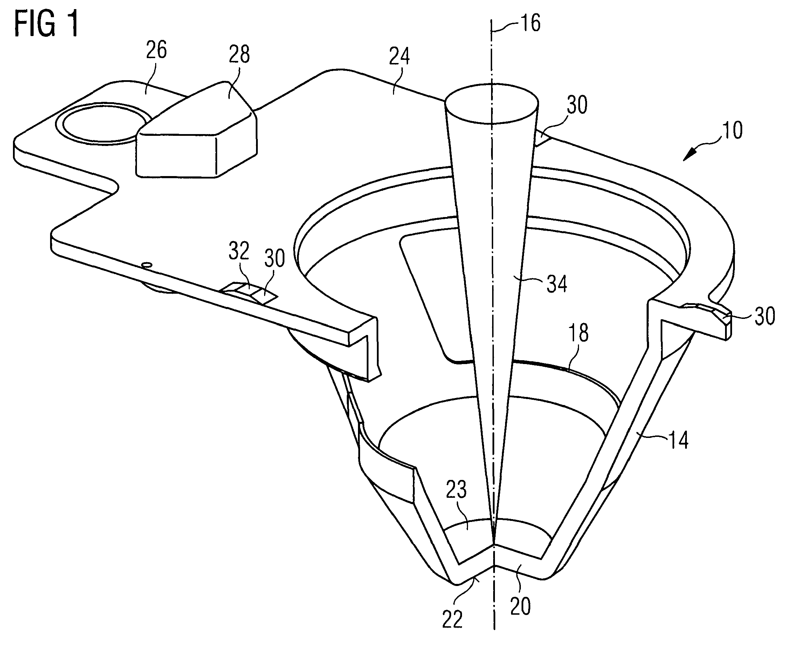

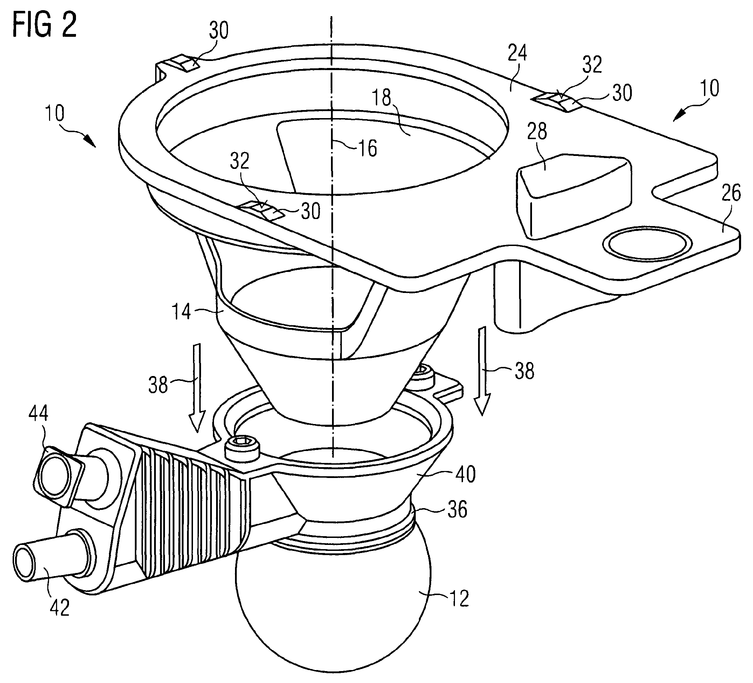

Es wird zunächst auf die Figuren 1 und 2 Bezug genommen. Dort ist eine allgemein mit 10 bezeichnete Schnittstelleneinheit erkennbar, welche zur Ankopplung an ein nicht näher dargestelltes Lasersystem dient und eine Positionierung und Referenzierung eines zu behandelnden humanen Auges 12 gegenüber dem Lasersystem ermöglicht. Die Schnittstelleneinheit 10 umfasst einen Abstandskegel 14 mit einer Kegelachse 16. Der Kegelmantel des Abstandskegels 14 ist im gezeigten Beispielfall von mehreren Durchbrechungen 18 durchsetzt; es versteht sich, dass der Kegelmantel alternativ als Vollmaterialmantel ausgeführt sein kann.

Wenn hier von einem Abstandskegel die Rede ist, so versteht es sich, dass weder die innenseitige noch die außenseitige Umfangsfläche des Kegels notwendigerweise exakt im mathematischen Sinne Kegelflächen bilden müssen. Stattdessen kann der Kegelmantel bei Voranschreiten in axialer Richtung ohne weiteres eine oder mehrere Abknickungen, Stufen oder Biegungen aufweisen. Insgesamt zeigt der Abstandskegel dennoch eine allgemeine Kegelgestalt, indem er sich ausgehend von einem seiner axialen Enden zunehmend in Richtung zum anderen axialen Ende radial aufweitet.

Es versteht sich, dass in einer Abwandlung ein zylindrischer oder andersartig geformter, innen hohler Abstandskörper anstelle des Abstandskegels 14 verwendet werden kann.

Am schmalen Ende des Abstandskegels 14 besitzt die Schnittstelleneinheit 10 eine hier planparallel ausgeführte Kontaktplatte 20, welche eine Kontaktfläche 22 zur Anlage an der Oberfläche des zu behandelnden Auges 12 bildet. Die Kontaktplatte 20 ist orthogonal zur Kegelachse 16 orientiert und wird in der Fachsprache aufgrund der Planität ihrer Kontaktfläche 22 üblicherweise als Applanationsplatte bezeichnet; sie ermöglicht eine Einebnung der Kornea des Auges 12.

Die zu der Kontaktfläche 22 gegenüberliegende Seite (d.h. augenabgewandte Seite) der Kontaktplatte 20 ist mit 23 bezeichnet.

Am breiten Ende des Abstandskegels 14 ist die Schnittstelleneinheit 10 zudem mit einem Montageflansch 24 ausgeführt, welcher sich rings um den Abstandskegel 14 erstreckt und radial von dem Abstandskegel 14 absteht. Auf einem Teil seiner Umfangserstreckung ist der Montageflansch 24 zu einer Griffplatte 26 erweitert, welche es einem Bediener ermöglicht, die Schnittstelleneinheit 10 zu greifen und in einen nicht näher dargestellten Einschub des Lasersystems radial einzuschieben. Die radiale Einschubtiefe kann durch einen an der Griffplatte 26 ausgebildeten Vorsprung 28 begrenzt sein, der mit einer nicht näher dargestellten radialen Anschlagfläche des Lasersystems zusammenwirkt. In dem Einschub ist die Schnittstelleneinheit 10 axial gegenüber dem Lasersystem festgelegt; gewünschtenfalls können geeignete Klemmelemente an dem Lasersystem vorgesehen sein, mittels welchen der Montageflansch 24 in dem Einschub festklemmbar ist.

An der Oberseite des Montageflansches 24 sind insgesamt drei in annährend gleichen Winkelabständen längs des Kegelumfangs verteilt angeordnete Positioniervorsprünge 30 vorgesehen, welche an ihrer Oberseite jeweils eine axial orientierte Positionierfläche 32 bilden. Die Positionierflächen 32 der Vorsprünge 30 gelangen beim Anbau der Schnittstelleneinheit 10 in axial positionierenden Eingriff mit einer axialen Anschlagfläche des Lasersystems, sodass über die gegenseitige Anlage der Positioniervorsprünge 30 an dieser Anschlagfläche eine feste axiale Positionierung der Schnittstelleneinheit 10 gegenüber dem Lasersystem gegeben ist.

Die Schnittstelleneinheit 10 stellt einen längs der Kegelachse 16 verlaufenden Durchgangsweg für die Laserstrahlung des Lasersystems 10 bereit, wie dies in Fig. 1 durch ein fokales Strahlenbündel 34 angedeutet ist. Der Durchgangsweg für die Laserstrahlung verläuft durch die Kontaktplatte 20 hindurch. Die Positionierflächen 32 der Positioniervorsprünge 30 bilden jeweils eine erste Positionierungsfläche im Sinne der Erfindung, dagegen bildet die Kontaktfläche 22 der Kontaktplatte 20 eine zweite Positionierungsfläche im Sinne der Erfindung.

Nachdem die Schnittstelleneinheit 10, insbesondere im Fall der Anwendung bei augenchirurgischen Behandlungen, aus Gründen der Hygiene häufig ein Einmalartikel ist, benötigen Anwender einen Vorrat an Schnittstelleneinheiten, um für jede Operation eine neue Schnittstelleneinheit einsetzen können. Eine Auswechslung der Schnittstelleneinheit soll freilich nicht dazu führen, dass das Lasersystem neu justiert werden muss, d. h. die z-Position des Strahlfokus neu referenziert werden muss. Dies stellt entsprechend hohe Anforderungen an die Fertigungsgenauigkeit der Schnittstelleneinheit 10, vor allem an den axialen (geometrischen) Abstand der Positionierflächen 32 von der Kontaktfläche 22 sowie an die Dicke der Kontaktplatte 20. Sowohl der geometrische Abstand der Positionierungsfläche 32 von der Kontaktfläche 22 als auch die axiale Dicke der Kontaktplatte 20 beeinflussen die effektive optische Weglänge der Schnittstelleneinheit 10 beim Durchgang des Strahlenbündels 34 durch die Schnittstelleneinheit 10.

Für eine hohe Fertigungspräzision und dementsprechend für eine hohe Präzision der optischen Eigenschaften der Schnittstelleneinheit 10 ist diese im gezeigten Beispielfall als einstückiger Schnittstellenkörper ausgebildet, d. h. der Abstandskegel 14 ist zusammen mit der Kontaktplatte 20 und dem Montageflansch 24 aus einem Stück hergestellt. Das Material dieses einstückigen Schnittstellenkörpers ist infolge der Transparenzanforderung der Kontaktplatte 20 ein für die Laserstrahlung transparentes Material. Vorzugsweise besitzt das Material des Schnittstellenkörpers im sichtbaren Wellenlängenbereich zudem eine hohe, farbneutrale Transparenz, um ein farbechtes, ausreichend helles Operationsfeld für den Arzt zu schaffen. Zur Herstellung eines vergleichsweise komplex geformten Gebildes wie der Schnittstelleneinheit 10 aus einem Stück eignet sich ein Kunststoffspritzverfahren, wobei die hohen Genauigkeitsanforderungen der Schnittstelleneinheit 10 besonders mit einem Spritzprägeverfahren erfüllbar sind. Unter Spritzprägen wird hierbei ein Verfahren verstanden, bei dem die Kunststoffschmelze in eine vergrößerte Kavität eingespritzt und in der anschließenden Prägephase mit beweglichen Werkzeugelementen komprimiert wird. Mit der Spritzprägetechnik lassen sich Bauteile in einer optischen Qualität herstellen, die selbst den hohen Anforderungen von Lasersystemen für ophthalmologische Anwendungen genügt.

Der für die Herstellung der Schnittstelleneinheit 10 verwendete Kunststoff ist zweckmäßigerweise biokompatibel. Geeignete Kunststoffe, die eine Zertifizierung als biokompatibel erhalten können, sind beispielsweise PMMA (Polymethylmethacrylat), Cyclo-Olefin-Polymere, Cyclo-Olefin-Copolymere sowie Polycarbonate. Beispiele für marktgängige erhältliche Materialien, die für die einstückige Herstellung der Schnittstelleneinheit 10 in einem Spritzprägeverfahren geeignet sind, sind Topas® von Topas Advanced Polymers und Zeonex® von Zeon Chemicals. Es versteht sich, dass eine Beschränkung auf eines dieser beispielhaften Materialien nicht beabsichtigt ist; jegliche Kunststoffmaterialien, die für ein Spritzprägeverfahren zugänglich sind und eine zumindest bei der Wellenlänge der verwendeten Laserstrahlung ausreichende Transmission zeigen und darüber hinaus ausreichend strahlungsstabil sind, sind grundsätzlich verwendbar.

Zur Vermeidung von Reflexionsverlusten empfiehlt es sich, den einstückig herstellten Schnittstellenkörper zumindest an einer oder beiden Plattenseiten der Kontaktplatte 20 mit einer reflexionsmindernden Beschichtung zu versehen.

Im Einsatz wird die Schnittstelleneinheit 10 entsprechend der Darstellung der Fig. 2 axial an eine zuvor auf das Auge 12 aufgesetzte und dort in nicht näher dargestellter Weise durch Saugkraft fixierte Saugringeinheit 36 angenähert, wie durch zwei Richtungspfeile 38 angedeutet. Dabei taucht der Abstandskegel 14 in einen Einführtrichter 40 der Saugringeinheit 36 ein und wird hierdurch gegenüber den Saugringeinheit 36 zentriert. Zwischen der Schnittstelleneinheit 10 und der Saugringeinheit 36 kann eine Saugkammer begrenzt sein, deren Evakuierung zu einem Ansaugen der Schnittstelleneinheit 10 an die Saugringeinheit 36 und damit zu einer gegenseitigen Fixierung dieser beiden Komponenten führt. Im Zuge des Eintauchens der Schnittstelleneinheit 10 in den Einführtrichter 40 der Saugringeinheit 36 kann die Kontaktplatte 20 mit ihrer Kontaktfläche 22 in Anlage an der Augenobertläche gelangen; es ist alternativ vorstellbar, dass im voll eingetauchten Zustand der Schnittstelleneinheit 10 die Kontaktplatte 20 noch nicht an dem Auge 12 anliegt und zur Herstellung des Anlagekontakts zwischen Auge 12 und Kontaktplatte 20 erst der Raum dazwischen evakuiert werden muss.

Die Saugringeinheit 36 ist im gezeigten Beispielfall mit zwei Anschlussstutzen 42, 44 ausgeführt, welche jeweils dem Anschluss einer nicht näher darstellten Schlauchleitung zur Verbindung mit einem Pumpsystem dienen. Jeder der Anschlussstutzen 42, 44 ist über ein internes Wegesystem mit jeweils einer Saugkammer der Saugringeinheit 36 verbunden, sodass diese beiden Saugkammern getrennt voneinander evakuierbar sind.

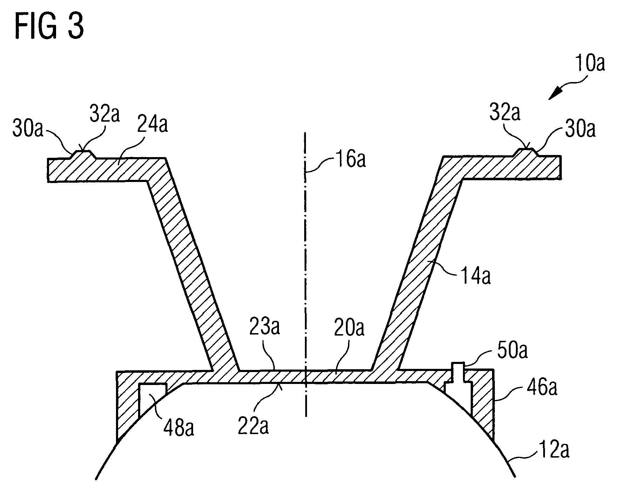

Bei dem in Fig. 3 gezeigten Ausführungsbeispiel sind gleiche oder gleichwirkende Komponenten mit gleichen Bezugszeichen wie zuvor versehen, jedoch ergänzt durch einen Kleinbuchstaben. Zur Vermeidung unnötiger Wiederholungen wird auf das vorstehend im Zusammenhang mit der Erläuterung der Figuren 1 und 2 Gesagte verwiesen, soweit sich nachstehend nichts anderes ergibt.

Die Schnittstelleneinheit 10a gemäß dem Ausführungsbeispiel der Fig. 3 ist von einem einstückig hergestellten Schnittstellenkörper gebildet, der nicht nur den Abstandskegel 14a, die Kontaktplatte 20a und den Montageflansch 24a bildet, sondern auch einen Saugring 46a mit einer zur Augenoberfläche hin offenen, der Ansaugung des Saugrings 46a an das Auge 12a dienenden Saugkammer 48a. Die Saugkammer 48a ist hier als Ringkammer ausgeführt, die längs ihres gesamten Ringumfangs zur Augenoberfläche hin offen ist und mit einem schematisch bei 50a angedeuteten Evakuierungsanschluss verbunden ist, an den eine nicht näher dargestellte Schlauchleitung zur Verbindung der Schnittstelleneinheit 10a mit einem Pumpsystem anschließbar ist. Die Schnittstelleneinheit 10a vereinigt somit die Funktionen eines Saugrings zur Fixierung des Auges 12a, der Applanation der Kornea des Auges 12a sowie der axialen Positionierung des Auges 12a gegenüber dem Lasersystem. Für die Herstellung der Schnittstelleneinheit 10a gilt das zuvor Gesagte; sie ist aus einem transparenten und biokompatiblen Kunststoffmaterial in einem Spritzverfahren hergestellt, insbesondere in einem Spritzprägeverfahren. Im Rahmen dieses Spritzverfahrens ist es vorstellbar, für den Saugring 46a ein anderes Material als für die verbleibenden Teile der Schnittstelleneinheit 10a, insbesondere den Abstandskegel 14a, die Kontaktplatte 20a und den Montageflansch 24a, zu verwenden. Auf diese Weise kann ein einstückiger Schnittstellenkörper hergestellt werden, der dennoch Bereiche besitzt, die aus jeweils unterschiedlichem Werkstoff bestehen. Alternativ ist es selbstverständlich möglich, die gesamte Schnittstelleneinheit 10a aus demselben Werkstoff zu fertigen.

## Patentansprüche

1. Kombination eines Lasersystems und einer Schnittstelleneinheit (10) zur Positionierung eines zu behandelnden humanen Auges gegenüber dem Lasersystem, wobei die Schnittstelleneinheit mindestens eine erste Positionierungsfläche (32) zur Positionierung der Schnittstelleneinheit gegenüber dem Lasersystem sowie eine zweite Positionierungsfläche (22) zur Anlage an dem Auge aufweist, wobei die Schnittstelleneinheit einen durch die zweite Positionierungsfläche hindurch verlaufenden Weg für die Strahlung des Lasersystems bereitstellt,
wobei die Schnittstelleneinheit einen sowohl die mindestens eine erste Positionierungsfläche als auch die zweite Positionierungsfläche bildenden, einstückig hergestellten Schnittstellenkörper umfasst, wobei der Schnittstellenkörper einen den Weg umschließenden Abstandskegel (14) sowie ein am schmalen Ende des Abstandskegels vorgesehenes Kontaktelement (20) zur Anlage an dem Auge aufweist,
**dadurch gekennzeichnet, dass**
der Schnittstellenkörper am breiten Ende des Abstandskegels mit einem radial von dem Abstandskegel abstehenden Montageflansch (24) ausgeführt ist, welcher sich rings um den Abstandskegel erstreckt und auf einem Teil seiner Umfangserstreckung zu einer Griffplatte (26) erweitert ist,
wobei das Lasersystem einen Einschub bereitstellt, in welchen die Schnittstelleneinheit mit dem Montageflansch radial einschiebbar ist und in welchem die Schnittstelleneinheit axial gegenüber dem Lasersystem festgelegt ist.

2. Kombination nach Anspruch 1, wobei der Schnittstellenkörper aus einem für ein Spritzverfahren, vorzugsweise Spritzprägungsverfahren, geeigneten Material, insbesondere Kunststoffmaterial hergestellt ist.

3. Kombination nach Anspruch 2, wobei das Kunststoffmaterial ein Cyclo-Olefin-Copolymer, ein Cyclo-Olefin-Polymer, Polymethylmethacrylat oder Polycarbonat umfasst.

4. Kombination nach einem der Ansprüche 1 bis 3, wobei zumindest ein Teil des Schnittstellenkörpers im sichtbaren Wellenlängenbereich transparent ist.

5. Kombination nach einem der vorhergehenden Ansprüche, wobei der Schnittstellenkörper verschiedene Bereiche besitzt, die aus jeweils unterschiedlichem Werkstoff bestehen.

6. Kombination nach einem der vorhergehenden Ansprüche, wobei zumindest ein die beiden Positionierungsflächen umfassender, zusammenhängender Abschnitt des Schnittstellenkörpers aus demselben Werkstoff besteht.

7. Kombination nach einem der vorhergehenden Ansprüche, wobei das Kontaktelement (20) eine augenzugewandte Kontaktfläche (22) aufweist, die plan, konkav, konvex oder zum Rand hin abgerundet ausgeführt ist.

8. Kombination nach Anspruch 7, wobei das Kontaktelement (20) auf seiner von der Kontaktfläche (22) abgewandten Seite (23) plan oder mit einer Freiformfläche ausgeführt ist.

9. Kombination nach einem der Ansprüche 1 bis 8, wobei das Kontaktelement (20) auf seiner augenzugewandten Seite (22) oder/und seiner augenabgewandten Seite (23) mit einer reflexionsmindernden Beschichtung versehen ist.

10. Kombination nach einem der Ansprüche 1 bis 9, wobei der Schnittstellenkörper mindestens einen zum Auge hin zumindest teilweise offenen Evakuierungsraum (48a) zur Saugfixierung des Schnittstellenkörpers an dem Auge aufweist.

11. Kombination nach Anspruch 10, wobei ein die beiden Positionierungsflächen umfassender, zusammenhängender erster Abschnitt des Schnittstellenkörpers aus einem anderen Werkstoff besteht als ein den Evakuierungsraum bildender zweiter Abschnitt des Schnittstellenkörpers.

## Claims

1. Combination of a laser system and an interface unit (10) for positioning a human eye to be treated relative to the laser system, wherein the interface unit has at least one first positioning surface (32) for positioning the interface unit relative to the laser system and also a second positioning surface (22) for bearing against the eye, wherein the interface unit provides a path passing through the second positioning surface for the radiation of the laser system,
wherein the interface unit comprises an integrally produced interface body that forms both the at least one first positioning surface and the second positioning surface, wherein the interface body has a spacer cone (14) enclosing the path and also a contact element (20) for bearing against the eye, said contact element being provided at the narrow end of the spacer cone,
**characterized in that**
the interface body at the wide end of the spacer cone is embodied with a mounting flange (24) projecting radially from the spacer cone, said mounting flange extending all around the spacer cone and being expanded on part of its circumferential extent to form a gripping plate (26),
wherein the laser system provides an insert into which the interface unit is radially insertable by the mounting flange and in which the interface unit is fixed axially relative to the laser system.

2. Combination according to Claim 1, wherein the interface body is produced from a material, in particular plastics material, suitable for an injection method, preferably injection compression moulding method.

3. Combination according to Claim 2, wherein the plastics material comprises a cycloolefin copolymer, a cycloolefin polymer, polymethyl methacrylate or polycarbonate.

4. Combination according to any of Claims 1 to 3, wherein at least part of the interface body is transparent in the visible wavelength range.

5. Combination according to any of the preceding claims, wherein the interface body has different regions consisting of respectively different materials.

6. Combination according to any of the preceding claims, wherein at least one continuous section of the interface body which comprises the two positioning surfaces consists of the same material.

7. Combination according to any of the preceding claims, wherein the contact element (20) has a contact surface (22) facing the eye and embodied in a planar fashion, concavely, convexly or in a manner rounded towards the edge.

8. Combination according to Claim 7, wherein the contact element (20), on its side (23) facing away from the contact surface (22) is embodied in a planar fashion or with a freeform surface.

9. Combination according to any of Claims 1 to 8, wherein the contact element (20) is provided with a reflexion-reducing coating on its side (22) facing the eye and/or its side (23) facing away from the eye.

10. Combination according to any of Claims 1 to 9, wherein the interface body has at least one evacuation space (48a) which is at least partly open towards the eye and which serves for the suction fixing of the interface body on the eye.

11. Combination according to Claim 10, wherein a continuous first section of the interface body which comprises the two positioning surfaces consists of a different material from a second section of the interface body which forms the evacuation space.

## Revendications

1. Combinaison d'un système laser et d'une unité d'interface (10) pour le positionnement, par rapport au système laser, d'un oeil humain à traiter, l'unité d'interface présentant au moins une première surface de positionnement (32) pour le positionnement de l'unité d'interface par rapport au système laser ainsi qu'une deuxième surface de positionnement (22) destinée à s'appliquer contre l'oeil, l'unité d'interface fournissant un trajet pour le faisceau du système laser, s'étendant à travers la deuxième surface de positionnement,
l'unité d'interface comprenant un corps d'interface fabriqué d'une seule pièce, constituant à la fois l'au moins une première surface de positionnement et la deuxième surface de positionnement, le corps d'interface présentant un cône d'espacement (14) entourant le trajet ainsi qu'un élément de contact (20) prévu au niveau de l'extrémité étroite du cône d'espacement et destiné à s'appliquer contre l'oeil,
**caractérisée en ce que**
le corps d'interface est réalisé au niveau de l'extrémité large du cône d'espacement avec une bride de montage (24) faisant saillie radialement depuis le cône d'espacement, qui s'étend tout autour du cône d'espacement et qui est élargie sur une partie de son étendue périphérique pour former une plaque de préhension (26),
le système laser fournissant un passage d'insertion dans lequel l'unité d'interface peut être insérée radialement avec la bride de montage et dans lequel l'unité d'interface est fixée axialement par rapport au système laser.

2. Combinaison selon la revendication 1, dans laquelle le corps d'interface est fabriqué en un matériau approprié pour un procédé de moulage par injection, de préférence un procédé de moulage par injection-compression, en particulier en un matériau en plastique.

3. Combinaison selon la revendication 2, dans laquelle le matériau en plastique comprend un copolymère cyclo-oléfinique, un polymère cyclo-oléfinique, un polyméthylméthacrylate ou un polycarbonate.

4. Combinaison selon l'une quelconque des revendications 1 à 3, dans laquelle au moins une partie du corps d'interface est transparente dans la plage de longueurs d'onde visible.

5. Combinaison selon l'une quelconque des revendications précédentes, dans laquelle le corps d'interface possède différentes régions qui se composent à chaque fois d'un matériau différent.

6. Combinaison selon l'une quelconque des revendications précédentes, dans laquelle au moins une portion continue du corps d'interface, comprenant les deux surfaces de positionnement, se compose du même matériau.

7. Combinaison selon l'une quelconque des revendications précédentes, dans laquelle l'élément de contact (20) présente une surface de contact tournée vers l'oeil (22) qui est réalisée sous forme plane, concave, convexe ou arrondie vers le bord.

8. Combinaison selon la revendication 7, dans laquelle l'élément de contact (20) est réalisé sur son côté (23) opposé à la surface de contact (22) sous forme plane ou avec une surface de forme libre.

9. Combinaison selon l'une quelconque des revendications 1 à 8, dans laquelle l'élément de contact (20) est pourvu sur son côté tourné vers l'oeil (22) et/ou sur son côté opposé à l'oeil (23) d'un revêtement anti-reflets.

10. Combinaison selon l'une quelconque des revendications 1 à 9, dans laquelle le corps d'interface présente au moins un espace d'évacuation (48a) ouvert au moins en partie vers l'oeil pour la fixation par aspiration du corps d'interface sur l'oeil.

11. Combinaison selon la revendication 10, dans laquelle une première portion continue du corps d'interface comprenant les deux surfaces de positionnement, se compose d'un autre matériau qu'une deuxième portion du corps d'interface formant l'espace d'évacuation.
